(19) Europäisches Patentamt
European Patent Office
Office européen des brevets

(11) Veröffentlichungsnummer: **0 395 192 A2**

(12) # EUROPÄISCHE PATENTANMELDUNG

(21) Anmeldenummer: **90250108.9**

(51) Int. Cl.5: **C07D 401/12, //C07D221/20**

(22) Anmeldetag: **27.04.90**

Weitere Erfinder : 20. Czuczor, Krüdy Gy. u. 12. II/5 H-4440 Tiszavasvari HU 21. Kovacs, Tancsics, u. 11/a, H-4440 Tiszavasvari HU.

(30) Priorität: **28.04.89 HU 203589**

(43) Veröffentlichungstag der Anmeldung:
**31.10.90 Patentblatt 90/44**

(84) Benannte Vertragsstaaten:
**BE CH DE ES LI**

(71) Anmelder: **ALKALOIDA VEGYESZETI GYAR**
**Kabay János ut 27-29**
**H-4440 Tiszavasvári(HU)**

(72) Erfinder: **Korodi, Ferenc, Dipl.-Chem.**
**Elmunkas u. 9. II./3**
**H-4440 Tiszavasvari(HU)**
Erfinder: **Köhegyi, Imre, Dipl.-Chem.**
**Elmunkas u. 11. II./4**
**H-4440 Tiszavasvari(HU)**
Erfinder: **Cziaky, Zoltan, Dipl.-Chem.**
**Kelp I. u. 1/b, 1/4.**
**H-4440 Tiszavasvari(HU)**
Erfinder: **Simon, Csaba, Dipl.-Chem.**
**Kelp I. u. 3/d fsz.**
**H-4440 Tiszavasvari(HU)**
Erfinder: **Frank, Laszlo, Dr. Biol.**
**Kabay J.u. 17-19. III/10**
**H-4440 Tiszavasvari(HU)**
Erfinder: **Hyiri, Laszlo**
**Kelp I. u. 3/b, IV/9.**
**H-4440 Tiszavasvari(HU)**
Erfinder: **Galamb, Vilmos, Dipl.-Chem. Dr.**
**Kelp I. u. 3/c, fsz.**
**H-4440 Tiszavasvari(HU)**
Erfinder: **Repasi, Janos, Dipl.-Chem. Dr.**
**Ifjusag u. 1/4**
**H-4440 Tiszavasvari(HU)**
Erfinder: **Jaszberenyi, Csaba, Dipl.-Chem. Dr.**
**Veszpremi u. 15**
**H-4440 Tiszavasvari(HU)**
Erfinder: **Bene, Dipl.-Chem.**
**Ifjusag u. 1/7**
**H-4440 Tiszavasvari(HU)**
Erfinder: **Ondi, Sandor**
**Kelp I. u. 3/c, 1/4**
**H-4440 Tiszavasvari(HU)**
Erfinder: **Barkanyi, Mrs.**
**Kelp I. u. 3/a**
**H-4440 Tiszavasvari(HU)**
Erfinder: **Terebes, Mrs.**
**Kinizsi u. 6/b**
**H-4440 Tiszavasvari(HU)**
Erfinder: **Flaskar, Mrs.**
**Kabay J. u. 25-27. IV/9**
**H-4440 Tiszavasvari(HU)**
Erfinder: **Korik, Piroska Baloghne**
**Csokonai u. 1**
**H-4450 Tiszalök(HU)**
Erfinder: **Mocsar, Maria**
**Sarvasi Pal u.8 1/4**
**H-4031-Debrecen(HU)**
Erfinder: **Tamas, Mrs.**
**Dozsa György u. 49**
**H-4440 Tiszavasvari(HU)**
Erfinder: **Fabian, Mrs.**
**Elmunkas u. 11.IV/13.**
**H-4440 Tiszavasvari(HU)**
Erfinder: **Juhasz, Mrs.**
**Vizmü u. 14,**
**H-4440 Tiszavasvari(HU)**

(74) Vertreter: **Maikowski, Michael, Dipl.-Ing. Dr. et al**
**Patentanwälte Maikowski & Ninnemann**
**Xantener Strasse 10**
**D-1000 Berlin 15(DE)**

(54) **Verfahren zur Herstellung von 8-(4-(4-Pyrimidin-2-yl-Piperazinyl)-Butyl)-8-Asaspiro(4.5)Dekan-7,9-Dion (Buspiron).**

EP 0 395 192 A2

(57) Die Erfindung betrifft ein Verfahren zur Herstellung von 8-[4-(4-Pyrimidin-2-yl-1-piperazinyl)-butyl]-8-azaspiro[4.5] dekan-7,9-dion durch Umsetzen von 8-(4-Piparazin-1-yl-butyl)-8-azaspiro[4.5]dekan7,9-dion mit 2-Chlorpyrimidin. Für das Verfahren ist charakteristisch, daß das Azaspiro[4.5]-dekan-7,9-dion-Derivat in einem inerten polaren Lösung)mittel, vorzugsweise Chloroform, Äthanol, Propanol, Butanol oder Dimethylformamid bei einer Temperatur von 50-150 °C, vorzugsweise 80-120 °C, in Gegenwart eines Säurebindemittels, oder aber bei 0-80 °C, vorzugsweise 60-70 °C, in Gegenwart eines Überschusses eines basischen Reagens, vorzugsweise Triäthylamin oder Pyridin, mit Chlorpyrimidin umgesetzt wird.

# VERFAHREN ZUR HERSTELLUNG VON 8-[4-(4-PYRIMIDIN-2-YL-1-PIPERA-ZINYL)-BUTYL]-8-AZASPIRO[4.5]-DEKAN-7,9-DION (BUSPIRON

Die Erfindung betrifft ein verbessertes Verfahren zur Herstellung des anxiolytisch wirksamen 8-[4-(4-Pyrimidin-2-yl-1-piperazinyl)-butyl]-8-azaspiro[4.5]dekan-7,9-dion der Formel I

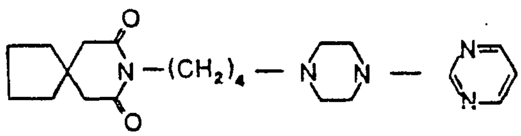

durch Umsetzen von 8-(4-Piperazin-1-yl-butyl)-8-azaspiro[4.5]-dekan-7,9-dion der Formel IV

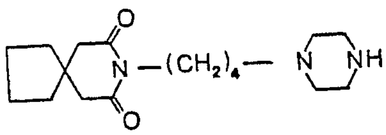

mit 2-Chlorpyrimidin der Formel V

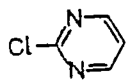

wobei die Verbindung der Formel IV durch Umsetzen von 8-(4-Brombutyl)-8-azaspiro[4.5]dekan-7,9-dion der Formel III

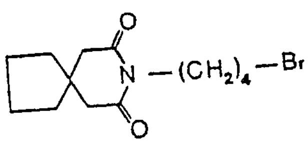

mit Piperazin, und die Verbindung der Formel III durch Alkylieren von 8H-8-Azaspiro[4.5]dekan-7,9-dion der Formel II

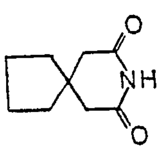

mit 1,4-Dibrombutan hergestellt wird.

Diese Reaktionsfolge ist aus der DE 31 49 011 bekannt. Sie wird dort zur Herstellung von radioaktiv markiertem Buspiron verwendet. Da das Ziel die laboratoriumsmäßige Herstellung einer für pharmakologische Versuche geeigneten Verbindung war, wurde offensichtlich auf eine besondere Verfahrensökonomie kein Gewicht gelegt. Die Gesamtausbeute der aufgeführten Reaktionen beträgt weniger als 10 %.

Ziel der Erfindung war die Verbesserung des Verfahrens durch Änderung der Verfahrensbedingungen.

Die Erfindung beruht auf der Erkenntnis, daß die Umsetzung der Piperazinverbindung der Formel IV als nukleophile Substitution dadurch gefördert werden kann, daß man als Lösungsmittel Verbindungen verwendet, die selber über ein freies Elektronenpaar verfügen, deshalb das freie Elektronenpaar der Piperazinverbindung nicht solvatieren und deshalb eine höhere Reaktionsbereitschaft gewährleisten. Überraschenderweise läßt sich dadurch nicht nur die Reaktionstemperatur vermindern, sondern auch die Reaktionszeit wesentlich verkürzen.

Die Verbindung der Formel IV wird aus 8-(4-Brombutyl)-8-azaspiro[4.5]dekan-7,9-dion der Formel III hergestellt. Diese Verbindung wird gemäß DE 31 49 011 mit Piperazin in Toluol in der Wärme umgesetzt. Erfindungsgemäß wird nun statt des Toluols ein polares Lösungsmittel eingesetzt, und das Piperazin wird in einem ungewöhnlich hohen Überschuß verwendet.

Die Verbindung der Formel III, das 8-(4-Brombutyl)-8-azaspiro[4.5]dekan-7,9-dion, wird durch Alkylieren von 8H-8-Azaspiro[4.5]dekan-7,9-dion (3,3-Tetramethylenglutarimid) der Formel II hergestellt. Erfindungsgemäß wird diese Reaktion in Gegenwart eines Säurebindemittels (wie Natriumhydrid, Kaliumcarbonat, Triäthylamin, Natriumhydroxyd, Natriummethylat) in einem inerter Lösungemittel (zum Beispiel Chloroform, Dichlormethan, Äthanol, Propanol, Butanol, Dimethylsulfoxyd, Dimethylformamid) mit einem 1-10fachen, vorzugsweise 2-5fachen Überschuß an 1,4-Dibrombutan bei 0-80 °C, vorzugsweise 10-20 °C, vorgenommen. Die Verbindung III entsteht dabei mit hoher Selektivität in ausgezeichneter Ausbeute. Sie braucht für die anschließenden Umsetzungen nicht gereinigt zu werden. Der Überschuß des 1,4-Dibrombutans kann zurückgewonnen und erneut verwendet werden.

Die Verbindung der Formel III wird in Gegenwart eines Säurebindemittels, zum Beispiel Triäthylamin, Kaliumcarbonat), in einem inerten polaren Lösungsmittel (zum Beispiel Chloro form, Äthanol, Propanol, Butanol, Dimethylformamid) bei 50-150 °C, vorzugsweise 80-120 °C, mit dem 1-30fachen, vorzugsweise 8-10-fachen Überschuß an Piperazin umgesetzt, wobei die Verbindung IV in hoher Ausbeute entsteht. Sie wird durch Überführen in eines ihrer gut kristallisierenden Säureadditionssalze (zum Beispiel Hydrochlorid, Hydrosulfat) gereinigt. Der Überschuß des Piperazins kann zurückgewonnen und erneut verwendet werden.

Die Verbindung IV wird mit 2-Chlorpyrimidin zum Endprodukt, dem 8-[4-(4-Pyrimidin-2-yl-1-piperazinyl)-butyl]-8-azaspiro[4.5]-dekan-7,9-dion (Buspiron), umgesetzt. Dies geschieht dadurch, daß man

a) die Verbindung IV oder deren mit einer Mineralsäure gebildetes Salz in Gegenwart eines Säurebindemittels (zum Beispiel Kaliumcarbonat, Triäthylamin) in einem inerten polaren Lösungsmittel (z.B. Chloroform, Äthanol, n-Butanol, Dimethylformamid) bei 50-150 °C, vorzugsweise 80-120 °C, mit etwa der äquivalenten Menge 2-Chlorpyrimidin umsetzt, oder

b) die Verbindung IV oder deren mit einer Mineralsäure gebildetes Salz in einem gleichzeitig als Lösungsmittel dienenden Säurebindemittel, vorzugsweise Pyridin oder Triäthylamin, bei 0-80 °C, vorzugsweise 60-70 °C, mit etwa der äquimolaren Menge 2-Chlorpyrimidin umsetzt.

Das Verfahren wird an den folgenden Beispielen näher erläuter

<u>Beispiel 1</u>

8-(4-Brombutyl)-8-azaspiro[4.5]dekan-7,9-dion (III)

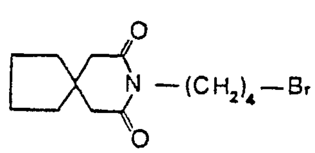

Ein Gemisch aus 60 ml Dimethylformamid und 30 ml 4M methanolischer Natriummethylat-Lösung wird unter Kühlen und Rühren bei Raumtemperatur tropfenweise mit der Lösung von 16,7 g (0,1 Mol) 8H-8-Azaspiro[4.5]dekan-7,9-dion in 60 ml Dimethylformamid versetzt. Das Gemisch wird auf 5-10 °C gekühlt und bei dieser Temperatur eine Stunde lang gerührt. Dann werden 54,0 g (0,25 Mol) 1,4-Dibrombutan zugesetzt. Das sich auf Raumtemperatur erwärmende Gemisch wird 2 Stunden lang gerührt und dann in 100 ml Eis wasser gegossen. Die organische Phase wird abgetrennt, die wässrige Phase wird mit 50 ml Chloroform extrahiert. Die vereinigten organischen Phasen werden über Natriumsulfat getrocknet und dann eingedampft. Man erhält 27,8 g 8-(4-Brombutyl)-8-azaspiro-[4.5]dekan-7,9-dion in Form eines blaßgelben, zähen Öles. Ausbeute: 92,0 %.
NMR: 1,5-2,0 (m, 12H], 2,6 (s, 4H), 3,42 (t, 2H), 3,8 (t , 2H).

<u>Beispiel 2</u>

8-(4-Piperazin-1-yl-butyl)-8-azaspiro[4.5]dekan-7,9-dion-dihydrochlorid (IV.2HCl)

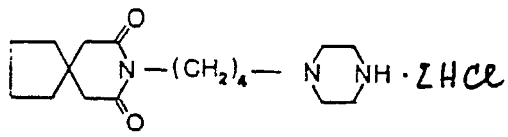

Ein Gemisch aus 27,8 g (0,092 Mol) 8-(4-Brombutyl)-8-azaspiro[4.5]deka-7,9-dion (Beispiel 1), 79,1 g (0,92 Mol) Piperazin und 13,8 g (0,1 Mol) pulverisiertes Kaliumcarbonat in 500 ml n-Butanol wird unter Rühren 2 Stunden lang am Rückflußkühler gekocht. Dann wird auf Raumtemperatur abgekühlt, die anorganischen Salze werden abfiltriert, das Filtrat wird eingedampft. Man erhält das Hydrochlorid, indem man das rohe Produkt in 70 ml Äthanol auflöst und die Lösung mit 20 ml 10 n salzsaurem Äthanol versetzt. Das Gemisch wird auf 0 °C gekühlt, die weißen Kristalle werden abfiltriert und mit Äthanol gewaschen. Man erhält 29,4 g (83 %) 8-(4-Piperazin-1-yl-butyl)-8-azaspiro[4.5]dekan-7,9-dion-dihydrochlorid, das bei 240-242 °C schmilzt.

Beispiel 3

Buspiron-HCl (I)

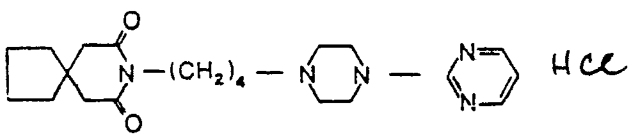

Ein Gemisch aus 9,5 g (0,025 Mol) 8-(4-Piperazin-1-yl-butyl-8-azaspiro[4.5]dekan-7,9-dion-dihydrochlorid, 2,86 g (0,025 Mol) 2-Chlorpyrimidin, 7,6 g (0,075 Mol) Triäthylamin und 125 ml n-Butanol wird unter Rühren 2 Stunden lang am Rückfluß gekocht. Das Reaktionsgemisch wird eingedampft, der Rückstand in 30 ml n HCl aufgelöst, die Lösung mit 10 ml Äther gewaschen, dann die wässrige Phase mit Aktivkohle geklärt und nach Alkalischmachen mit 5n Natronlauge die ausgefallene Substanz abfiltriert und mit Wasser gewaschen. Man erhält 7,85 g 8-[4-(4-Pyridin-2-yl-1-piperazinyl)-butyl]-8-azaspiro[4.5]dekan-7,9-dion (81,6 %), das bei 102-103 °C schmilzt.

Die Base wird in 20 ml Äthanol gelöst, die Lösung mit 2 ml 10n salzsaurem Äthanol versetzt, mit Aktivkohle geklärt und dann auf 5 °C gekühlt. Das ausgefallene Hydrochlorid wird abfiltriert. Man erhält 7,57 g Buspiron-dihydrochlorid (71,8 %), das bei 201-203 °C schmilzt. Dünnschichtchromatographische Untersuchungen zeigen, daß die Gsamtverunreinigung des Produktes höchstens 0,2 % beträgt.

Beispiel 4

Buspiron-HCl (I)

30,7 g (0,10 Mol) 8-(4-Piperazin-1-yl-butyl)-8-azaspiro[4.5]-7,9-dion werden zusammen mit 11,4 g (0,10 Mol) 2-Chlorpyrimidin und 180 ml Triäthylamin bei Raumtemperatur 72 Stunden lang oder bei 60-70 °C eine Stunde lang gerührt. Das Reaktionsgemisch wird eingedampft, der Eindampfrückstand in 120 ml 1n Salzsäure gelöst, die wässrige Phase mit Aktivkohle geklärt, mit 5n Natronlauge alkalisch gemacht, die Substanz wird abfiltriert und mit Wasser gewaschen. Man erhält 31,3 g (81,3 %) 8-[4-(4-Pyrimidin-2-yl-1-piperazinyl)-butyl]-8-azaspiro[4.5]dekan-7,9-dion, das bei 101-102 °C schmilzt. Die Herstellung des Hydrochlorids erfolgt auf die im Beispiel 3 angegebene Weise.

Der Vorteil des erfindungsgemäßen Verfahrens besteht darin, daß bei allgemein kürzeren Reaktionszeiten die Gesamtausbeute über 50 % beträgt, während die Gesamtausbeute der drei Schritte gemäß dem Stand der Technik unter 10 % liegt.

## Ansprüche

1. Verfahren zur Herstellung von 8-[4-(4-Pyrimidin-2-yl-1-piperazinyl)-butyl]-8-azaspiro[4.5]dekan-7,9-dion der Formel I

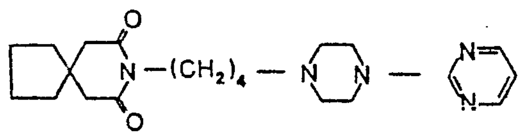

und seines Hydrochlorids durch Umsetzen von 8-(4-Piperazin-1-yl-butyl)-8-azaspiro[4.5]dekan-7,9-dion der Formel IV

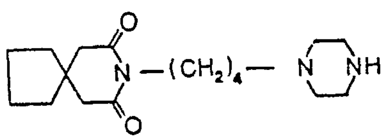

mit 2-Chlorpyrimidin der Formel V

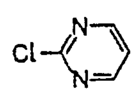

wobei die Verbindung der Formel IV durch Umsetzen von 8-(4-Brombutyl)-8-azaspiro[4.5]dekan-7,9-dion der Formel III

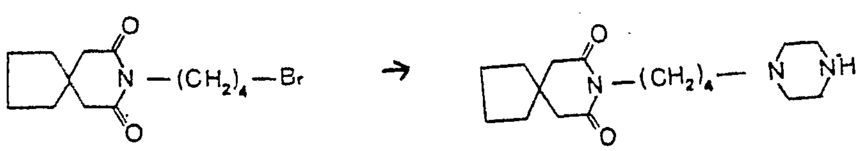

mit Piperazin, und die Verbindung der Formel III durch Alkylieren von 8H-8-Azaspiro[4,5]dekan-7,9-dion der Formel II

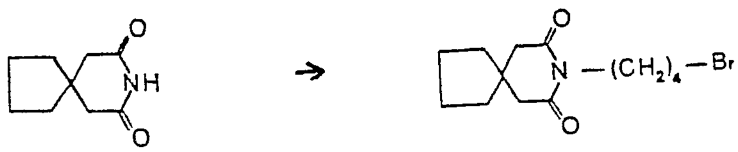

mit 1,4-Dibrombutan hergestellt wird,
dadurch **gekennzeichnet,** daß man
a) das 8-(4-Piperazin-1-yl-butyl)-8-azaspito[4.5]dekan-7,9-dion der Formel IV oder sein Säureadditionssalz in einem inerten polaren Lösungsmittel, vorzugsweise Chloroform, Äthanol, Propanol, Butanol oder Dimethylformamid, bei einer Temperatur von 50-150 °C, vorzugsweise 80- 120 °C in Gegenwart eines Säurebindemittels, oder aber bei 0-80 °C, vorzugsweise 60-70 °C, in Gegenwart eines Überschusses eines basischen Reagens, vorzugsweise Triäthylamin oder Pyridin, mit 2-Chlorpyrimidin der Formel V umsetzt und die erhaltene Base gewünschten falls in ihr Hydrochlorid überführt, oder
b) das 8-(4-Brombutyl)-8-azaspiro[4,5]dekan-7,9-dion der Formel III mit einem 2-30fachen, vorzugsweise 8-10fachen Überschuß an Piperazin umsetzt, gegebenenfalls in Gegenwart eines Säurebindemittels, in einem inerten Lösungsmittel wie Chloroform, Äthanol, Propanol, Butanol oder Dimethylformamid bei einer Temperatur zwischen 50 und 150 °C, vorzugsweise 80-120 °C,
dann das erhaltene 8-(4-Piperazin-1-yl-butyl)-8-azaspiro[4,5]dekan-7,9-dion der Formel IV oder sein Säureadditionssalz in einem inerten polaren Lösungsmittel, vorzugsweise Chloroform, Äthanol, Propanol, Butanol

oder Dimethylformamid, bei einer Temperatur von 50-150 °C, vorzugsweise 80-120 °C in Gegenwart eines Säurebindemittels,

oder aber bei 0-80 °C, vorzugsweise 60-70 °C, in Gegenwart eines Überschusses eines basischen Reagens, vorzugsweise Triäthylamin oder Pyridin, mit 2-Chlorpyrimidin der Formel V umsetzt und die erhaltene Base gewünschtenfalls in ihr Hydrochlorid überführt oder

c) das 8H-8-Azaspiro[4.5]dekan-7,9-dion der Formel II mit 1,4-Dibrombutan in Gegenwart eines Säurebindemittels, vorzugsweise Natriumhydrid, Kaliumcarbonat, Natriummethylat oder Natriumhydroxyd, in Gegenwart eines inerten Lösungsmittels, vorzugsweise Chloroform, Dichlormethan, Äthanol, Propanol, Butanol, Dimethylsulfoxyd oder Dimethylformamid, bei 0-80 °C, vorzugsweise bei 10-20 °C, alkyliert,

dann das erhaltene 8-(4-Brombutyl)-8-azaspiro[4,5]dekan-7,9-dion der Formel III mit einem 2-30fachen, vorzugsweise 8-10fachen Überschuß an Piperazin umsetzt, gegebenenfalls in Gegenwart eines Säurebinders, in einem inerten Lösungsmittel wie Chloroform, Äthanol, Propanol, Butanol oder Dimethylformamid bei einer Temperatur zwischen 50 und 150 °C, vorzugsweise 80-120 °C,

dann das erhaltene 8-(4-Piperazin-1-yl-butyl)-8-azaspiro[4,5]dekan-7,9-dion der Formel IV oder sein Säureadditionssalz in einem inerten polaren Lösungsmittel, vorzugsweise Chloroform, Äthanol, Propanol, Butanol oder Dimethylformamid, bei einer Temperatur von 50-150 °C, vorzugsweise 80-120 °C, in Gegenwart eines Säurebindemittels,

oder aber bei 0-80 °C, vorzugsweise 60-70 °C, in Gegenwart eines Überschusses eines basischen Reagens, vorzugsweise Triäthylamin oder Pyridin, mit 2-Chlorpyrimidin umsetzt und die erhaltene Base gewünschtenfalls in ihr Hydrochlorid überführt.

2. Verfahren zur Herstellung von 8-(4-Piperazin-1-yl-butyl)-8-azaspiro[4.5]dekan-7,9-dion der Formel IV und seiner Salze durch Umsetzen des 8-(4-Brombutyl)-8-azaspiro[4.5]dekan7,9-dions der Formel III mit Piperazin

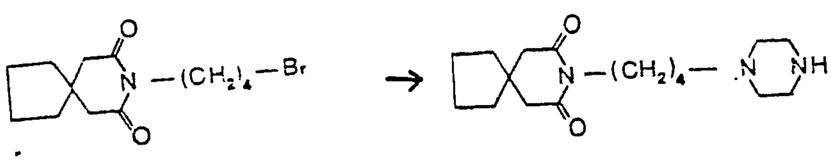

wobei die Verbindung der Formel III durch Alkylieren des 8H-8-Azaspiro[4.5]dekan-7,9-dions der Formel II erhalten wurde,

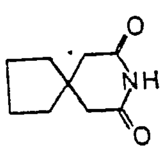

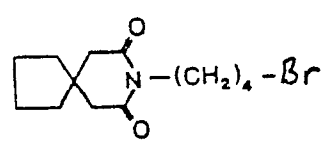

dadurch **gekennzeichnet,** daß man

a) das 8-(4-Brombutyl)-8-azaspiro[4.5]dekan-7,9-dion der Formel III mit einem 2-30fachen, vorzugsweise 8-10fachen Überschuß an Piperazin umsetzt, gegebenenfalls in Gegenwart eines Säurebindemittels, in einem inerten Lösungsmittel wie Chloroform, Äthanol, Propanol, Butanol oder Dimethylformamid bei einer Temperatur zwischen 50 und 150 °C, vorzugsweise 80-120 °C, und gewünschtenfalls die erhaltene Verbindung in ihr Salz überführt oder

b) das 8H-8-Azaspiro[4.5]dekan7,9-dion der Formel II mit 1,4-Dibrombutan in Gegenwart eines Säurebindemittels, vorzugsweise Natriumhydrid, Kaliumcarbonat, Natriummethylat oder Natriumhydroxyd in einem inerten Lösungsmittel, vorzugsweise Chloroform, Dichlormethan, Äthanol, Propanol, Butanol, Dimethylformamid bei einer Temperatur von 0-80 °C, vorzugsweise 10-20 °C, alkyliert und dann

das erhaltene 8-(4-Brombutyl)-8-azaspiro[4.5]dekan-7,8-dion der Formel III mit einem 2-30fachen, vorzugsweise 8-10-fachen Überschuß an Piperazin umsetzt, gegebenenfalls in Gegenwart eines Säurebinders, in einem inerten Lösungsmittel wie Chloroform, Äthanol, Propanol, Butanol oder Dimethylformamid bei Temperaturen zwischen 50 und 150 °C, vorzugsweise 80-120 °C, und gewünschtenfalls die erhaltene Verbindung IV zu ihrem Salz, vorzugsweise ihrem Hydrochlorid, umsetzt.

3. Verfahren zur Herstellung von 8-(4-Brombutyl)-8-azaspiro[4.5]dekan-7,8-dion der Formel III durch Alkylieren von 8H-8-Azaspiro[4.5]dekan-7,9 der Formel II mit 1,4-Dibrombutan, dadurch **gekennzeichnet,** daß man 8H-8-Azaspiro-[4.5]dekan-7,9-dion der Formel II nit 1,4-Dibrombutan in Gegenwart eines Säurebindemittels, vorzugsweise Natriumhydrid, Kaliumcarbonat, Natriummethylat oder Natriumhydroxyd, in einem inerten Lösungsmittel, vorzugsweise in Chloroform, Dichlormethan, Äthanol, Propanol, Butanol, Dimethylsulfoxyd bei Temperaturen zwischen 0 und 80 °C, vorzugsweise 10 -20 °C, alkyliert.